(19) Europäisches Patentamt
European Patent Office
Office européen des brevets

(11) Veröffentlichungsnummer : **0 036 834**
**B1**

(12) # EUROPÄISCHE PATENTSCHRIFT

(45) Veröffentlichungstag der Patentschrift : **07.12.83**

(51) Int. Cl.³ : **C 07 D403/12**, C 07 D209/44, C 09 B 57/04, C 09 B 26/02

(21) Anmeldenummer : **81810081.0**

(22) Anmeldetag : **09.03.81**

(54) **Verfahren zur Herstellung von Metallkomplexen von Isoindolinazinen.**

(30) Priorität : **13.03.80 CH 1975/80**

(43) Veröffentlichungstag der Anmeldung : **30.09.81 Patentblatt 81/39**

(45) Bekanntmachung des Hinweises auf die Patenterteilung : **07.12.83 Patentblatt 83/49**

(84) Benannte Vertragsstaaten : **CH DE FR GB IT LI**

(56) Entgegenhaltungen : **DE-A- 2 504 321**

(73) Patentinhaber : **CIBA-GEIGY AG**
**Patentabteilung Postfach**
**CH-4002 Basel (CH)**

(72) Erfinder : **Iqbal, Abul, Dr.**
**Im Schalengarten 1**
**CH-4107 Ettingen (CH)**
Erfinder : **Lienhard, Paul, Dr.**
**Kirschgartenstrasse 14**
**CH-4402 Frenkendorf (CH)**

**EP 0 036 834 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Metallkomplexen von Isoindolinazinen

In der GB-PS 1.467.595 ist die Herstellung von Azinen der Formel

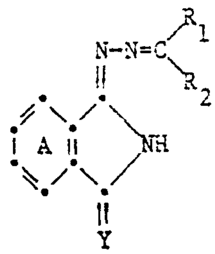

(I)

beschrieben, worin $R_1$ ein H-Atom, eine Alkyl- oder Arylgruppe, $R_2$ einen isocyclischen oder heterocyclischen Rest, beide mit einer zur Azomethingruppe benachbarten Hydroxy- oder Mercaptogruppe, Y den Rest einer methylenaktiven Verbindung, eines Aryl- oder Heteroarylamins bedeutet, und der Ring A nicht wasserlöslichmachende Substituenten aufweisen kann, durch Kondensation einer Verbindung der Formel

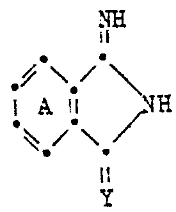

(Ia)

mit einem Hydrazon der Formel

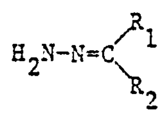

(III)

Dabei erhält man die Verbindung der Formel (I) zwar in guter Ausbeute, die daraus erhaltenen 1 : 1-Nickelkomplexe ergeben jedoch in Kunststoffen und Lacken Färbungen von unbefriedigender Reinheit und ungenügenden Echtheiten. Die Kondensation der Verbindung der Formel (Ia) mit dem Hydrazon der Formel (III) in Gegenwart von Nickelsalzen führt ebenfalls zu keinen befriedigenden Ergebnissen.

Es wurde nun gefunden, dass man die 1 : 1-Metallkomplexe von Azinen der Formel (I) in ausgezeichneter Ausbeute und Reinheit erhält, wenn man eine Verbindung der Formel

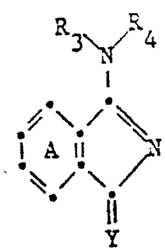

(II)

worin $R_3$ ein H-Atom oder einen Alkylrest, $R_4$ einen Alkyl-, Aryl- oder Heteroarylrest oder den Rest der Formel

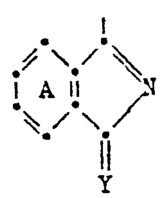

(IIa)

bedeuten, oder $R_3$ und $R_4$ zusammen mit dem N-Atom einen 5- oder 6-gliedrigen Heteroring darstellen,

und Y den Rest der Formel

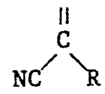

worin R für eine Cyangruppe, eine Alkoxycarbonyl-, Alkylcarbamoyl- oder Alkanoylgruppe mit 2-6 C, eine Benzoyl-, Carbamoyl-, Thiocarbamoyl- oder Sulfamoylgruppe, eine Benzylcarbamoylgruppe, eine gegebenenfalls durch Halogenatome oder Alkylgruppen mit 1-4 C substituierte Phenylsulfamoyl, Phenylsulfonylgruppe oder eine Gruppe der Formel

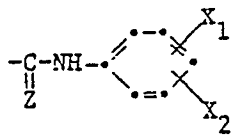

oder

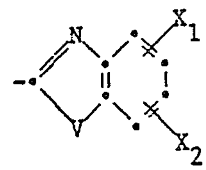

steht, worin Z ein Sauerstoff- oder Schwefelatom, $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylengruppen substituierte Phenoxy-, Phenylcarbamoyl- oder Phenylsulfamoylgruppe und $X_2$ ein H-, Chlor- oder Bromatom, eine Alkyl- oder Alkoxygruppe mit 1-4 C und V O, S oder -NH bedeuten mit einem Hydrazon der Formel

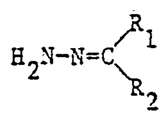

(III)

worin $R_1$ und $R_2$ die angegebene Bedeutung haben, in Gegenwart einer ein Uebergangsmetall abgebenden Verbindung in einem polaren organischen Lösungsmittel bei Temperaturen zwischen 100°-200 °C umsetzt.

Die als Ausgangsstoffe zu verwendenden Aminoisoindolenine der Formel (II) können als Substituenten im Benzolrest A Halogenatome, beispielsweise 2-4 Chloratome, 1-2 Alkyl- oder Alkoxygruppen mit 1-4 C, eine Phenyl-, Phenoxy- oder Nitro-, eine Alkanoylaminogruppe mit 2-6 C oder eine Benzoylaminogruppe enthalten, sind jedoch vorzugsweise im Rest A unsubstituiert.

Sofern der Rest $R_3$ ein H-Atom bedeutet ist für die Verbindung (II) auch die tautomere Isoindolinform möglich.

Sofern $R_3$ und $R_4$ Alkylreste bedeuten, dann vorzugsweise solche mit 1-6 C steht $R_4$ für einen Arylrest, dann vorzugsweise für einen gegebenenfalls durch Chloratome, Alkyl- oder Alkoxygruppen mit 1-4 C substituierten Phenylrest.

Die Verbindung der Formel (II) erhält man nach bekanntem Verfahren durch Kondensation eines Aminoisoindolenins der Formel

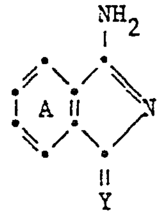

(V)

mit einem Amin der Formel $HNR_3R_4$.

Als Beispiele von Aminen seien genannt :

Alkylamine wie Methylamin, Aethylamin, n-Propylamin, Isopropylamin, n-Butylamin, n-Hexylamin, n-Oktylamin, n-Decylamin oder Laurylamin.

Bevorzugt sind aromatische Amine, insbesondere Aminobenzole der Formel

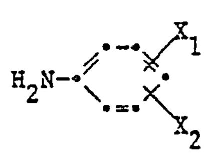

worin $X_1$ und $X_2$ die oben angegebene Bedeutung haben.

Aus der Reihe der heterocyclischen Amine seien die in der GB-PS 1.467.595 S. 6-7 genannten und ausserdem 2-Aminopyridin, 2-Amino-5-chlorpyridin, 2-Amino-4-hydroxychinolin und 2-Amino-4,5-dimethyl-thiazol erwähnt.

Als Beispiele von Aminen der Formel $HNR_3R_4$, worin $R_3$ und $R_4$ zusammen mit dem N-Atom einen 5- oder 6-gliedrigen Heteroring darstellen, seien genannt : Pyrrolidin, Morpholin, Piperidin etc.

Da der Aminrest bei der Kondensation abgespalten wird, empfiehlt sich die Verwendung möglichst preisgünstiger Amine.

Die Verbindung der Formel (V) erhält man durch Kondensation des entsprechenden 1-Amino-3-imino-isoindolenins mit einer methylenaktiven Verbindungen der Formel $NCCH_2R$, worin R die oben angegebene Bedeutung hat.

Als Beispiele seien die in der GB-PS 1.467.595, Seite 7 aufgeführten Acetonitrile, ferner Cyanessigsäure-o-chlorphenyl-, p-chlorphenyl-, -m-chlorphenyl-, -m-methylphenyl-, -p-methylphenyl-, -3,4-dichlorphenyl-, -3,5-dimethylphenyl-, -3,4-dimethylphenyl-, -3-chlor-4-methylphenyl-, -o-methoxyphenyl-, -2,4-dimethoxyphenyl-, -2,5-dimethoxyphenyl-, p-acetylamino-phenyl-, p-benzoylaminophenyl-, -3-chlor-4-p-chlorbenzoylaminophenyl-, 4-carbamoylphenyl-, -4-sulfamoylphenyl-, -4-phenylazophenyl-, -4-phenoxyphenyl-, -p-nitrophenyl-, 3-trifluormethylphenyl-, oder -2-chlor-5-trifluormethylphenylamide, -2-Cyanmethyl-4-phenyl-, -4-p-nitrophenyl-, -4-fluorphenyl- oder -4-methylphenylthiazol erwähnt.

In den Hydrazonen der Formel (III) steht $R_1$ beispielsweise für einen Phenylrest, vorzugsweise aber für ein H-Atom oder eine Alkylgruppe mit 1-4 C, insbesondere die Methylgruppe. Sofern $R_2$ einen isocyclischen Rest bedeutet, dann beispielsweise einen Phenyl- oder Naphthalinrest, vorzugsweise aber einen ein- oder zweikernigen Heteroring.

Die Hydrazone der Formel (III), erhält man nach bekanntem Verfahren durch Umsetzen einer Oxoverbindung der Formel

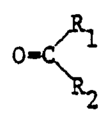

bzw. deren Aldimin mit Hydrazinhydrat. Von besonderem Interesse sind Aldehyde oder Ketone der Formel

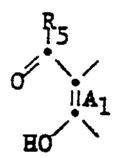

worin $R_5$ im H-Atom oder eine Methylgruppe $A_1$ einen Naphthalinrest oder einen 5- oder 6-gliedrigen Heteroring bedeutet, der in α- oder γ-Stellung zum C-Atom, an dem die Hydroxygruppe sitzt ein O-, S- oder insbesondere N-Atom aufweist und gegebenenfalls noch ein weiteres N-Atom im Ring und gegebenenfalls einen ankondensierten Benzolring und/oder einen weiteren Heteroring enthält.

Bevorzugt sind Oxoverbindungen der Formel

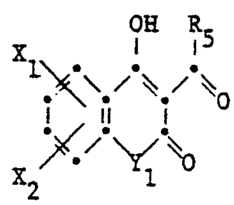

worin $R_5$, $X_1$ und $X_2$ die oben angegebenen Bedeutungen haben und $Y_1$ ein O- oder S-Atom oder eine NH-Gruppe bedeutet.

Ebenfalls von besonderem Interesse sind die Verbindungen der nachstehenden Formel

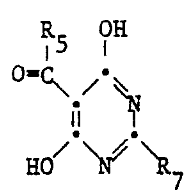

worin $R_5$ die angegebene Bedeutung hat, $R_6$ eine Cyan, Alkoxycarbonyl- oder Carbamoylgruppe und $R_7$ ein Wasserstoffatom, eine Alkyl-, Aryl- oder Hydroxygruppe bedeuten, oder

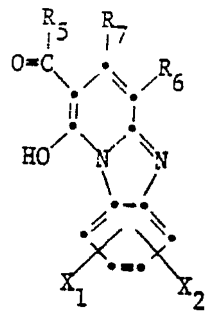

worin $X_1$, $X_2$, $R_5$, $R_6$ und $R_7$ die oben angegebene Bedeutung haben, oder

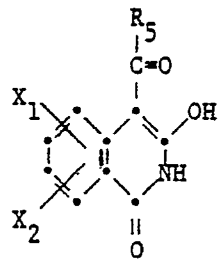

worin $X_1$, $X_2$ und $R_5$ die oben angegebene Bedeutung haben, ferner Hydroxynaphthaldehyde der Formel

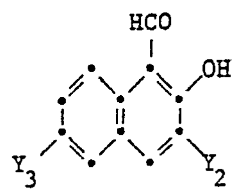

worin $Y_2$ ein H-Atom, eine Carboxy- oder Carbamoylgruppe, eine Alkoxycarbonyl- oder Alkylcarbamoylgruppe enthaltend 2-6 C-Atome, eine gegebenenfalls im Phenylrest durch Halogenatome, Alkyl- oder Alkoxygruppen, enthaltend 1-4 C-Atome substituierte Phenylcarbamoylgruppe, $Y_3$ ein H- oder Halogenatom, eine Methoxy-, Nitro- oder Cyangruppe bedeuten, oder Pyrazole der Formel

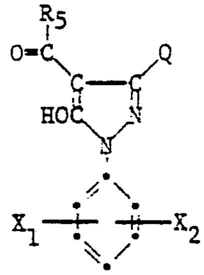

worin $R_5$, $X_1$ und $X_2$ die oben angegebene Bedeutung haben, Q eine Methylgruppe oder eine Alkoxycarbonylgruppe enthaltend 2-5 C-Atome oder eine Carbamoylgruppe bedeutet.

Als Beispiele seien die in der GB-PS 1.467.595 aufgeführten Aldehyde und Ketone genannt.

Als metallabgebende Mittel verwendet man vorzugsweise die Salze des Zinks, Cadmiums, Mangans, Kobalts, Eisens, insbesondere aber des Kupfers und des Nickels bzw. Mischungen solcher Metalle. Man verwendet zweckmässig die Formiate, Acetate oder Stearate dieser Metalle.

Die Umsetzung des Aminoindolenins der Formel (II) mit dem Hydrazon der Formel (III) erfolgt in einem polaren Lösungsmittel, insbesondere einem solchen hydrophiler Natur, beispielsweise einem Amid wie Dimethylformamid, Formamid, Dimethylacetamid oder N-Methylpyrrolidon, ferner Dimethylsulfoxid, Acetonitril oder einem Alkohol wie beispielsweise Aethylcellosolve. Es kann auch ein Gemisch polarer Lösungsmittel verwendet werden.

Die Umsetzungstemperatur liegt zweckmässig zwischen 100-200 °C.

Die Isolierung des erhaltenen Metall-Komplexes erfolgt wie üblich durch Filtration. Das Nutschgut wird mit Lösungsmittel gut gewaschen. Es wird in ausgezeichneter Ausbeute und Reinheit erhalten und kann ohne weitere Reinigung in feinverteilter Form zum Färben von hochmolekularem organischem

Material verwendet werden, beispielsweise von Celluloseäthern und -estern, wie Aethylcellulose, Acetylcellulose, Nitrocellulose, Polyamiden bzw. Polyurethanen oder Polyestern, natürlichen Harzen oder Kunstharzen, z. B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyäthylen, Polypropylen, Polyacrylnitril, Polyacrylsäureester, thermoplastische oder härtbare Acrylharze, Gummi, Casein, Silikon und Silikonharzen, einzeln oder in Mischungen. Die erwähnten hochmolekularen Verbindungen können als plastische Massen, Schmelzen oder in Form von Spinnlösungen, Lacken oder Druckfarben vorliegen. Je nach Verwendungszweck erweist es sich als vorteilhaft, die Pigmente als Toner oder in Form von Präparaten zu verwenden.

Das Pigment kann in der Form, wie es in der Synthese anfällt, oder in leicht gemahlener Form eingesetzt werden und liefert dann opake Ausfärbungen. Man kann es aber auch einer intensiveren Mahlung unterziehen und erhält dann transparente Ausfärbungen, wie beispielsweise farbstarke Metalleffektlackierungen.

Anreibungen der Pigmente in Lacken zeichnen sich durch ein günstiges Fliessverhalten aus.

Die erhaltenen Färbungen beispielsweise in Kunststoffen, Fasern und Lacken zeichnen sich durch grosse Farbstärke, hohe Farbtonreinheit, gute Dispergierbarkeit, gute Ueberlackier-, Migrations-, Hitze-, Licht- und Wetterechtheit sowie durch einen guten Glanz aus.

Gegenüber den nach dem Verfahren der GB-PS 1.467.595 erhältlichen Pigmenten zeichnen sich die erfindungsgemäss erhaltenen durch höhere Reinheit und bessere Echtheiten, insbesondere bessere Migrations-, Ueberlackier-, Licht-, Wetter- und Hitzebeständigkeit aus.

Beispiel 1

0,65 g (0,003 Mol) 3-Acetyl-2,4-dihydroxychinolinhydrazon (hergestellt durch Umsetzen des 3-Acetyl-2,4-dihydroxychinolins mit Hydrazinhydrat in siedendem Aethanol) sowie 0,78 g Nickelacetat $4H_2O$ (0,003 15 Mol) werden in 40 ml N-Methylpyrrolidon gelöst und auf 60 °C erwärmt. Das Gemisch wird anschliessend mit 1,2 g (0,003 Mol) 1-(Cyan-benzimidazolylmethylen)-3-N-p-nitrophenylimino-isoindolin (hergestellt durch Umsetzen des 1-(Cyan-benzimidazolylmethylen)-3-imino-isoindolins mit p-Nitroanilin in Dimethylformamid) versetzt und während 1 1/2 Stunden bei 150-155 °C gerührt. Man filtriert heiss (80 °C) und wäscht das Nutschgut mit N-Methylpyrrolidon und Aethanol nach. Nach der Trocknung bei 80 °C am Vakuum erhält man 1,5 g (92,3 % d. Th.) eines roten Metallkomplexes der Zusammensetzung $C_{28}H_{17}N_7O_2Ni$ und der Formel

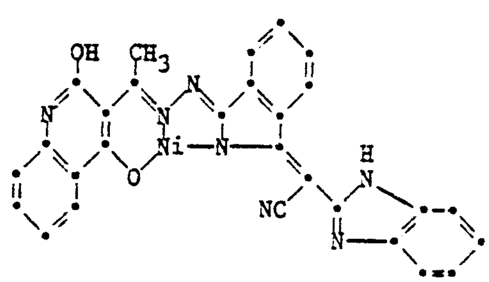

Mikroanalyse : $C_{28}H_{17}N_7O_2Ni$ (Molgewicht 542,20)

| | | | | |
|---|---|---|---|---|
| berechnet | 62,03 % C | 3,16 % H | 18,08 % N | 10,83 % Ni |
| gefunden | 62,10 % C | 3,20 % H | 18,30 % N | 10,80 % Ni |

Er färbt Kunststoffe und Lacke in reinen roten Tönen von ausgezeichneten Echtheiten.

Beispiele 2-7

Setzt man im obigen Beispiel anstelle von 1-(Cyan-benzimidazolylmethylen)-3-N-p-nitrophenylimino-isoindolin die nachfolgend genannten 1-(Cyan-benzimidazolylmethylen)-3-N-arylimino-isoindoline enthaltend als Aryliminogruppen die folgenden Reste ein :

Beispiel 3 : Phenylimino
Beispiel 4 : p-Tolylimino
Beispiel 5 : p-Methoxyphenylimino
Beispiel 6 : p-Chlorphenylimino
Beispiel 7 : p-Acetylphenylimino

so erhält man bei analoger Arbeitsweise ebenfalls in hoher Ausbeute und Reinheit den gleichen Metall-Komplex wie oben.

Beispiel 8

1,09 g (0,005 Mol) 3-Acetyl-2,4-dioxychinolin-hydrazon sowie 1,31 g Nickelacetat $4H_2O$ (0,005 Mol + 5 %) werden in 40 ml N-Methylpyrrolidon suspendiert und auf 60 °C erwärmt. Das Gemisch versetzt man anschliessend mit 2,17 g (0,005 Mol) 1-(Cyan-p-chlorphenylcarbamoylmethylen)-3-N-p-chlorphenylimino-isoindolin der Formel

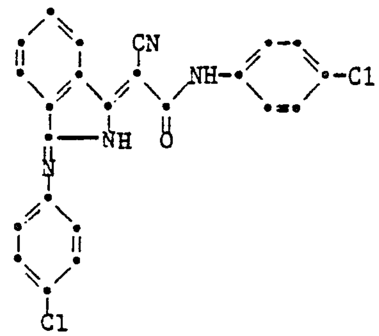

Es wird auf 150-155 °C erhitzt und während 1 1/2 Stunden bei derselben Temperatur gerührt. Man filtriert bei 80 °C und wäscht das Nutschgut mit N-Methylpyrrolidon und Aethanol nach. Nach Trocknen bei 80 °C unter Vakuum erhält man 2,22 g (76 % d. Th.) eines roten Metallkomplexes der Formel

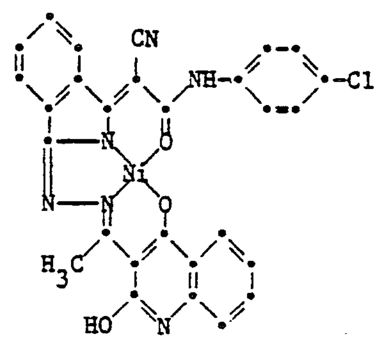

der Kunststoffe und Lacke in reinen roten Tönen von ausgezeichneter Hitze-, Licht-, Wetter- und Migrationsechtheit färbt.

Mikroanalyse : $C_{28}H_{17}ClN_6O_3Ni$ (Molgewicht 579,65)

| | | | | | |
|---|---|---|---|---|---|
| berechnet | 58,02 % C | 2,96 % H | 14,50 % N | 6,12 % Cl | 10,13 % Ni |
| gefunden | 57,70 % C | 3,30 % H | 14,30 % N | 5,90 % Cl | 9,70 % Ni |

Beispiel 9

Ersetzt man im Beispiel 8 1-(Cyan-p-chlorphenyl-carbamoyl-methylen)-3-N-p-chlorphenylimino-isoindolin durch das 1-(Cyan-p-chlorphenylcarbamoylmethylen-3-imino-isoindolin, so erhält man in 60 %iger Ausbeute einen ebenfalls roten Nickelkomplex mit der gleichen Zusammensetzung wie der gemäss Beispiel 8 erhaltene, die damit erhaltenen Färbungen in PVC, Lacken und Polyolefinen sind jedoch weniger rein und von geringerer Licht-, Wetter- und Hitzebeständigkeit. Ein ähnlich mangelhaftes Metallkomplex-Pigment erhält man auch dann, wenn man nach Beispiel 78 der GB-PS 1.467.595 arbeitet.

Beispiel 10

Verwendet man im Beispiel 8 anstelle des 1-(Cyan-p-chlorphenylcarbamoylmethylen)-3-N-p-chlorphenylimino-isoindolins das 3-N-morpholinoderivat, so erhält man bei analoger Versuchdurchführung und Aufarbeitung einen roten Nickelkomplex, der die gleiche Zusammensetzung, Reinheit und Echtheiten wie der gemäss Beispiel 8 erhaltene aufweist.

Beispiel 11

Verwendet man im Beispiel 8 anstelle des 1-(Cyan-p-chlorphenylcarbamoylmethylen)-3-N-p-chlor-

phenylimino-isoindolins das entsprechende 3-N-piperidino-Derivat, so erhält man unter sonst identischen Reaktionsbedingungen einen ebenfalls reinen roten Nickelkomplex mit der gleichen Zusammensetzung, der gleichen Licht-, Wetter- und Hitzebeständigkeit wie der gemäss Beispiel 8 erhaltene.

Beispiele 12-37

In der nachfolgenden Tabelle sind weitere Nickelkomplexe der Formel

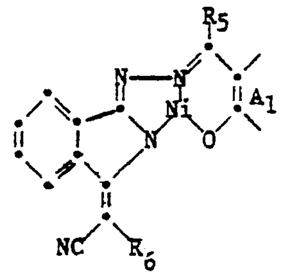

(VI)

aufgeführt, (der Einfachheit halber wird nur eine der möglichen isomeren oder tautomeren Formen berücksichtigt), die man erhält, wenn man nach den Angaben der Beispiele 1 und 8 ein Hydrazon der Formel

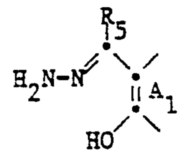

(VII)

worin $R_5$ die in Kolonne 2 und $A_1$ die in Kolonne 3 angegebene Bedeutung hat, mit einem 1-Methin-3-arylimino-isoindolin der Formel

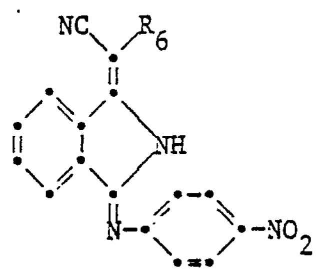

(VIII)

kondensiert, worin $R_6$ die in Kolonne 4 angegebene Bedeutung hat, Kolonne 5 gibt die Nuance der Färbung in Polyvinylchlorid.

Die Herstellung der Ausgangsprodukte der Formel VII erfolgt durch Umsetzen der entsprechenden Formyl- oder Acetylverbindungen mit Hydrazinhydrat nach bekannten Verfahren. Die Verbindungen der Formel VIII werden ebenfalls nach bekanntem Verfahren durch Umsetzen des entsprechenden 1-Cyanmethylen-3-imino-isoindolins mit p-Nitroanilin gewonnen.

(Siehe Tabelle 1, Seite 9 ff.)

Tabelle 1

| Bei-spiel Nr. | $R_5$ | 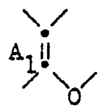 | $R_6$ | Nuance in PVC |
|---|---|---|---|---|
| 12 | H | 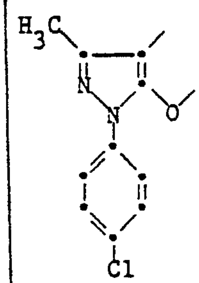 | 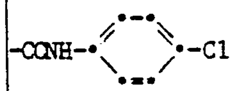 | rot |
| 13 | $CH_3$ | 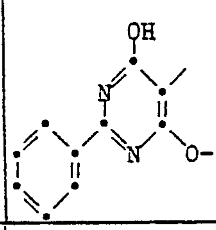 | 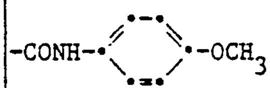 | rot |
| 14 | H | 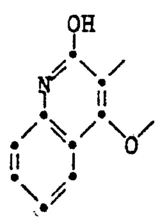 | 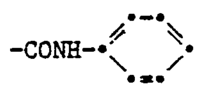 | orange |
| 15 | $CH_3$ | 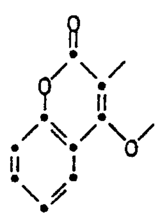 | 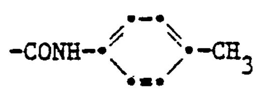 | orange |
| 16 | -do- | -do- | 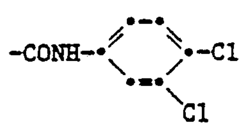 | gelb |
| 17 | -do- | -do- | 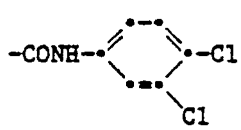 | orange |

(Fortsetzung)

| Beispiel Nr. | $R_5$ | 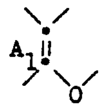 | $R_6$ | Nuance in PVC |
|---|---|---|---|---|
| 18 | -do- | 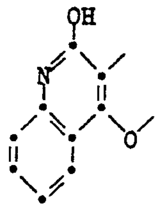 | 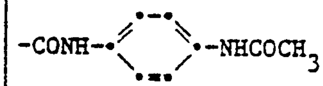 | rot |
| 19 | -do- | -do- | 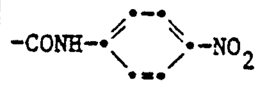 | rot |
| 20 | H | 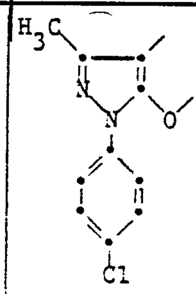 | 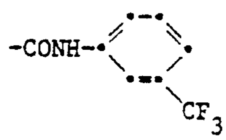 | scharlach |
| 21 | -do- | -do- | 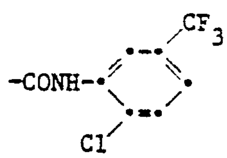 | rot |
| 22 | -do- | -do- | 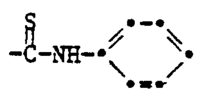 | rot |
| 23 | -do- | -do- | 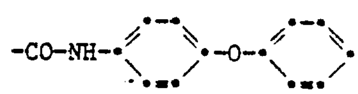 | rot |
| 24 | -do- | -do- | 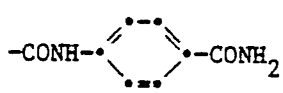 | scharlach |
| 25 | -do- | -do- | 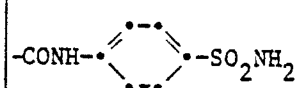 | orange |
| 26 | $CH_3$ | 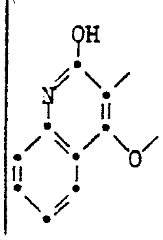 | 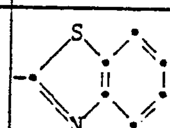 | rot |

(Fortsetzung)

| Bei-spiel Nr. | $R_5$ | 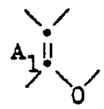 | $R_6$ | Nuance in PVC |
|---|---|---|---|---|
| 27 | $CH_3$ | 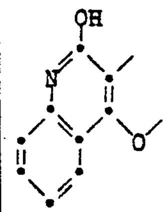 | 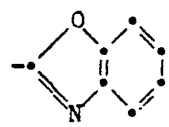 | orange |
| 28 | -do- | -do- | 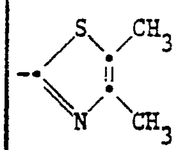 | orange |
| 29 | -do- | -do- | 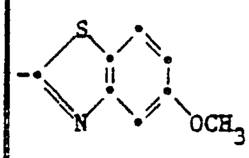 | rot |
| 30 | H | 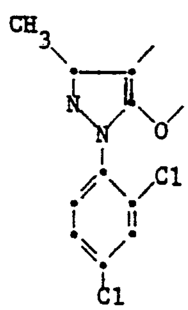 | -CONH- 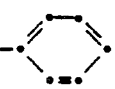 | rot |
| 31 | $CH_3$ | 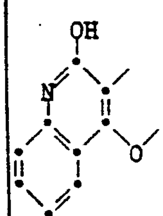 | 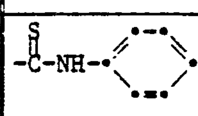 | rot |
| 32 | -do- | -do- | 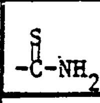 | rot |

(Fortsetzung)

| Bei-spiel Nr. | $R_5$ | 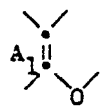 | $R_6$ | Nuance in PVC |
|---|---|---|---|---|
| 33 | $CH_3$ | 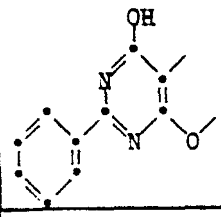 | 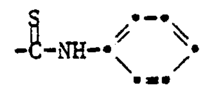 | rot |
| 34 | H | 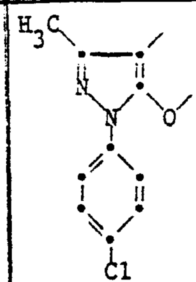 | 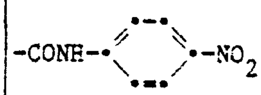 | orange |
| 35 | $CH_3$ | 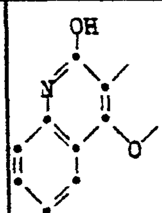 | 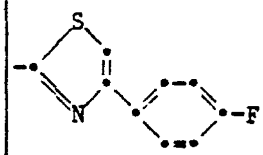 | rot |
| 36 | -do- | -do- | 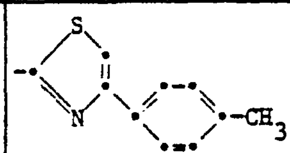 | rot |
| 37 | -do- | -do- | 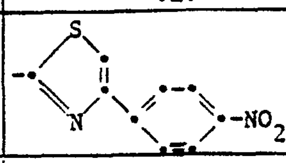 | rot |
| 38 | H | 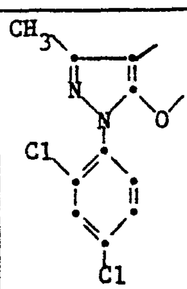 | 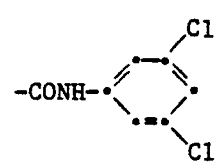 | rot |

Beispiel 39

17,1 g (0,06 Mol) 1-(Cyan-benzimidazolylmethylen)-3-imino-isoindolin, hergestellt aus 1,3-Diiminoisoindolin und Cyanmethylbenzimidazol, werden in 90 ml Dimethylformamid angeschlämmt und mit 7,14 ml n-Butylamin (0,072 Mol) versetzt. Das Gemisch wird auf 100 °C erwärmt und bei derselben Temperatur 3 Stunden lang gerührt. Anschliessend wird auf 5 °C abgekühlt und filtriert. Das Nutschgut wird mit wenig Dimethylformamid und Sprit gewaschen und über Nacht unter Vakuum bei 80 °C getrocknet. Man erhält 10,8 g (50,2 % d. Th.) der Verbindung der Formel

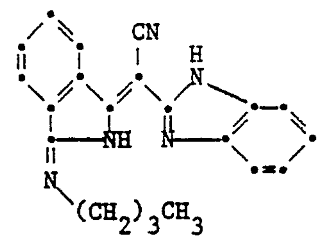

als oranges Pulver.

Mikroanalyse : $C_{21}H_{19}N_5$ Molgewicht 341,4

| | | | |
|---|---|---|---|
| berechnet | 73,9 % C | 5,61 % H | 20,5 % N |
| gefunden | 74,1 % C | 5,6 % H | 20,6 % N |

Beispiel 40

Setzt man im Beispiel 1 anstelle von 1-(Cyan-benzimidazolylmethylen)-3-N-p-nitrophenylimino-isoindolin das durch Umsetzen des 1-(Cyan-benzimidazolylmethylen)-3-imino-isoindolins mit n-Butylamin in Dimethylformamid nach Beispiel 39 hergestellte 1-(Cyan-benzimidazolylmethylen)-3-N-butylimino-isoindolin-Derivat ein, so erhält man bei analoger Versuchsdurchführung ebenfalls in hoher Reinheit den gleichen Metallkomplex wie im Beispiel 1.

Beispiel 41

16,25 g (0,05 Mol) 1-(Cyan-p-chlorphenylcarbamoylmethylen)-3-imino-isoindolin, hergestellt aus 1,3-Diiminoisoindolin und Cyanaceto-p-chloranilid, wird in 100 ml Dimethylformamid eingetragen. Anschliessend gibt man 6 ml einer 33 %igen alkoholischen Lösung von Methylamin hinzu, das Gemisch wird 1 1/4 h bei 80-90 °C gerührt, mit 30 ml Dimethylformamid verdünnt und weiteren 2 ml Methylamin-Lösung versetzt. Nachdem man das Gemisch weitere 5 h bei 90-100 °C hat ausreagieren lassen, wird das Reaktionsprodukt bei 80 °C abfiltriert, mit Dimethylformamid und Sprit gewaschen und über Nacht unter Vakuum bei 70 °C getrocknet. Man erhält 15,2 g (90,2 % d. Th.) der Verbindung der Formel

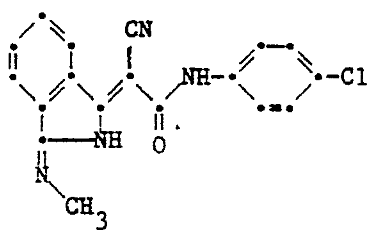

als gelbes Pulver.

Mikroanalyse : $C_{18}H_{13}ClN_4O$ Molgewicht.

| | | | | |
|---|---|---|---|---|
| berechnet | 64,20 % C | 3,89 % H | 16,64 % N | 10,53 % Cl |
| gefunden | 64,2 % C | 3,8 % H | 16,7 % N | 10,5 % Cl |

Beispiel 42

Verwendet man im Beispiel 8 anstatt 1-(Cyan-p-chlorphenylcarbamoylmethylen)-3-N-p-chlorphenylimino-isoindolin das nach Beispiel 41 aus Methylamin und 1-(Cyan-o-chlorphenylcarbamoylmethylen)-3-imino-isoindolin erhältliche 1-(Cyan-o-chlorphenylcarbamoylmethylen)-3-N-methylimino-isoindolin, so erhält man bei analoger Aufarbeitung das gleiche Metallkomplex-Pigment wie im Beispiel 8.

Beispiel 43

5,46 g (0,139 Mol) 1-(Cyan-p-Chlorphenylcarbamoylmethylen)-3-imino-5,6-dichlor-isoindolin, hergestellt aus 5,6-Dichlor-1,3-diiminoisoindolin und Cyanacet-p-chloranilid, werden in 40 ml Dimethylformamid mit 1,7 ml (0,174 2 Mol) n-Butylamin versetzt und anschliessend bei 100-105 °C 4 Stunden lang gerührt. Hierauf wird das Gemisch auf 10 °C abgekühlt und filtriert. Das Nutschgut wird mit wenig Dimethylformamid und Sprit nachgewaschen und über Nacht bei 70 °C unter Vakuum getrocknet. Man erhält 4,15 g (87,29 % d. Th.) der Formel

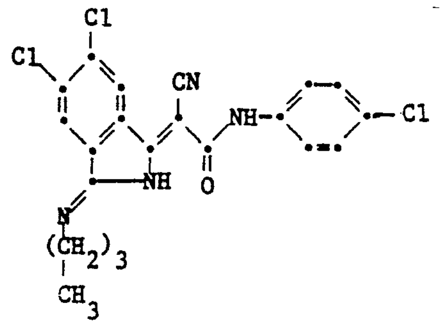

als gelbes Pulver.

Mikroanalyse : $C_{21}H_{17}N_4OCl_3$ Molgewicht 447,8

| | | | |
|---|---|---|---|
| berechnet | 56,3 % C | 3,83 % H | 12,5 % N |
| gefunden | 55,8 % C | 4,4 % H | 12,9 % N |

Beispiel 44

2,56 g des Zwischenproduktes aus Beispiel 43 werden zu einer auf 60 °C vorgewärmten Suspension von 1,3 g (0,006 Mol) 3-Acetyl-2,4-dioxychinolinhydrazon sowie 1,57 g (0,006 3 Mol) Nickelacetat $4H_2O$ in 40 ml N-Methylpyrrolidon hinzugegeben. Das Gemisch wird auf 150 °C erhitzt und während zwei Stunden bei derselben Temperatur gerührt. Anschliessend filtriert man bei 80 °C und wäscht das Nutschgut mit N-Methylpyrrolidon und Aethanol nach. Nach Trocknen bei 80 °C unter Vakuum erhält man 2,12 g (54,5 % d. Th.) eines roten Pigments der folgenden Struktur (nur eine der möglichen isomeren bzw. tautomeren Formen wurde berücksichtigt) :

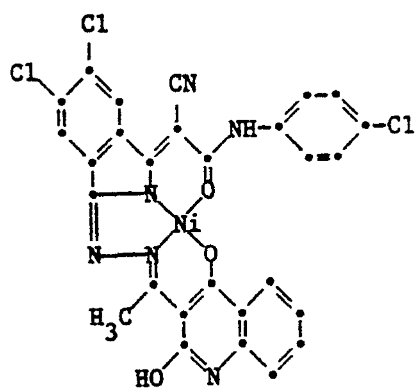

das Kunststoffe und Lacke in reinen roten Tönen von ausgezeichneter Hitze-, Licht-, Wetter- und Migrationsechtheit färbt.

Mikroanalyse : $C_{28}H_{15}Cl_3N_6O_3Ni$ Molgewicht 648,5

| | | | | | |
|---|---|---|---|---|---|
| berechnet | 51,86 % C | 2,33 % H | 16,40 % Cl | 12,96 % N | 9,05 % Ni |
| gefunden | 51,8 % C | 2,8 % H | 15,8 % Cl | 13,1 % N | 9,10 % Ni |

Beispiele 45-48

Verwendet man im Beispiel 8 anstatt 1-(Cyan-p-chlorphenylcarbamoylmethylen)-3-N-p-chlorphenylimino-isoindolin die nachfolgend genannten Indoline der Formel

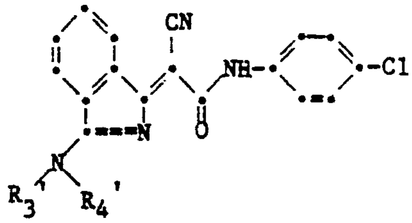

ein, so erhält man bei analoger Arbeitsweise ebenfalls in hoher Ausbeute und Reinheit den gleichen Metallkomplex wie im Beispiel 8.

Beispiel 45

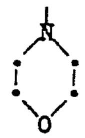

Beispiel 46

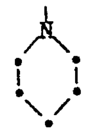

Beispiel 47

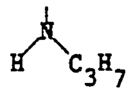

Beispiel 48

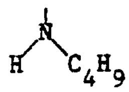

Beispiel 49

In einem Laboratoriums-Kneter von 250 Vol.-Teilen Inhalt legt man 25 Teile des gemäss Beispiel 8 hergestellten Pigments, 100 Teile feingemahlenes Natriumchlorid und 30 Teile Diacetonalkohol vor. Man knetet die Mischung während 5 Stunden unter Kühlung und trägt sie dann in 4 000 Vol.-Teile Wasser ein. Natriumchlorid sowie Diacetonalkohol gehen in Lösung, das Pigment fällt aus. Man filtriert die Suspension, wäscht das Nutschgut gründlich mit Wasser und trocknet es im Vakuumtrockenschrank bei 80°.

Beispiel 50

65 Teile stabilisiertes Polyvinylchlorid, 35 Teile Dioctylphthalat und 0,2 Teile des gemäss Beispiel 51 erhaltenen Pigments werden miteinander verrührt und dann auf einem Zweiwalzenkalander während 7 Minuten bei 140° hin- und hergewalzt. Man erhält eine orange-rot gefärbte Folie von sehr guter Licht- und Migrationsechtheit.

Beispiel 51

10 g Titandioxyd und 2 g des nach Beispiel 8 hergestellten Pigments werden mit 88 g einer Mischung von 26,4 g Kokosalkydharz, 24,0 g Melamin-Formaldehydharz (50 % Festkörpergehalt), 8,8 g Aethylenglykolmonomethyläther und 28,8 g Xylol während 48 Stunden in einer Kugelmühle vermahlen.

Wird dieser Lack auf eine Aluminiumfolie gespritzt, 30 Minuten bei Raumtemperatur vorgetrocknet und dann während 30 Minuten bei 120 °C eingebrannt, dann erhält man eine Rotlackierung, die sich bei guter Farbstärke durch eine sehr gute Ueberlackier-, Licht- und Wetterechtheit auszeichnet.

Beispiel 52

4 Teile des fein verteilten Pigments gemäss Beispiel 51 werden in 20 Teilen Lösungsmittel der folgenden Zusammensetzung eingerührt: 50 Teile Solvesso 150 (Gemisch aromatischer Kohlenstoffe), 15 Teile Butylacetat, 5 Teile Exkin II (Verlaufmittel auf Ketoximbasis), 25 Teile Methyl-Isobutylketon, 5 Teile Silikonöl (1 % in Solvesso 150).

Nachdem die vollständige Feinverteilung erreicht ist (je nach Art des Rührens in ca. 15-60 Minuten),

werden die Bindemittel zugesetzt, nämlich 48,3 Teile Baycryl L 530 (Acrylharz) (51 % in Xylol/Butanol 3 : 1) und 23,7 Teile Maprenal TTX (Melaminharz) (55 % in Butanol).

Nach kurzem Homogenisieren wird der Lack nach üblichen Methoden wie Spritzen und Tauchen oder speziell zur kontinuierlichen Beschichtung von Metallblechen im « Coil-Coating »-Verfahren appliziert und eingebrannt (Einbrennen 30 Minuten, 130°). Die erhaltenen roten Lackierungen zeichnen sich aus durch sehr guten Verlauf, hohen Glanz und ausgezeichnete Feinverteilung des Pigmentes, sowie durch ausgezeichnete Wetterechtheiten.

Beispiel 53

Verfährt man wie in Beispiel 51 beschrieben, fügt jedoch der Knetmasse 2,78 Teile Staybelite Resin (HERCULES) zu, so erhält man ein Pigment, enthaltend 10 % Harz, das sich auszeichnet durch leichtere Einarbeitbarkeit und bessere Verteilbarkeit.

**Ansprüche**

1. Verfahren zur Herstellung von 1 : 1-Metallkomplexen von Azinen der Formel

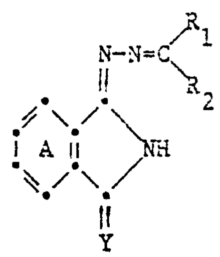

(I)

worin $R_1$ ein H-Atom, eine Alkyl- oder Arylgruppe, $R_2$ einen isocyclischen oder heterocyclischen Rest, beide mit einer zur Azomethingruppe benachbarten Hydroxy- oder Mercaptogruppe, Y den Rest der Formel

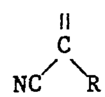

worin R für eine Cyangruppe, eine Alkoxycarbonyl-, Alkylcarbamoyl, oder Alkanoylgruppe mit 2-6 C, eine Benzoyl-, Carbamoyl-, Thiocarbamoyl- oder Sulfamoylgruppe, eine Benzylcarbamoylgruppe, eine gegebenenfalls durch Halogenatome oder Alkylgruppen mit 1-4 C substituierte Phenylsulfamoyl, Phenylsulfonylgruppe, oder eine Gruppe der Formel

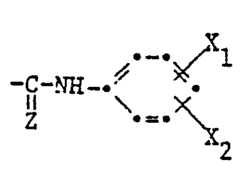

oder

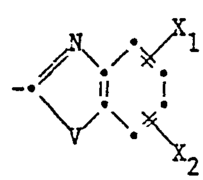

steht, worin Z ein Sauerstoff- oder Schwefelatom, $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylengruppen substituierte Phenoxy-, Phenylcarbamoyl- oder Phenylsulfamoylgruppe und $X_2$ ein H-, Chlor- oder Bromatom, eine Alkyl- oder Alkoxygruppe mit 1-4 C und V O, S oder -NH bedeuten, und der Ring A nicht wasserlöslichmachende Substituenten aufweisen kann, dadurch gekennzeichnet, dass man eine Verbindung der Formel

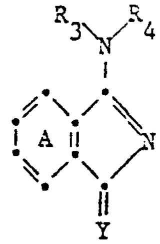

(II)

worin $R_3$ ein H-Atom oder einen Alkylrest, $R_4$ einen Alkyl-, Aryl- oder Heteroarylrest oder den Rest der Formel

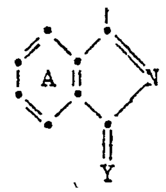

bedeuten, oder $R_3$ und $R_4$ zusammen mit dem N-Atom einen 5- oder 6-gliedrigen Heteroring darstellen, und Y die angegebene Bedeutung hat, mit einem Hydrazon der Formel

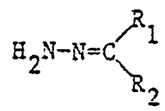

in Gegenwart einer ein Uebergangsmetall abgebenden Verbindung in einem polaren organischen Lösungsmittel bei Temperaturen zwischen 100° und 200 °C umsetzt.

2. Verfahren gemäss Anspruch 1, dadurch gekennzeichnet, dass man von einer Verbindung der Formel (II) ausgeht, worin R eine Carbamoyl- oder Thiocarbamoylgruppe oder eine Gruppe der Formel

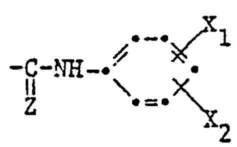

oder

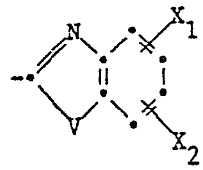

bedeutet, worin Z ein Sauerstoff- oder Schwefelatom ist, $X_1$ und $X_2$ die in Anspruch 1 angegebene Bedeutung haben.

3. Verfahren gemäss Anspruch 1, dadurch gekennzeichnet, dass man von einer Verbindung der Formel (II) ausgeht, worin $R_3$—N—$R_4$ den Rest der Formel

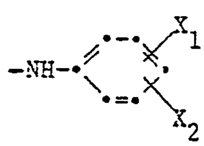

bedeutet, worin $X_1$ und $X_2$ die im Anspruch 1 angegebene Bedeutung haben.

4. Verfahren gemäss Anspruch 1, dadurch gekennzeichnet, dass man von einem Hydrazon der Formel

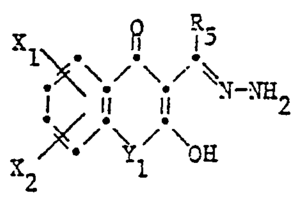

ausgeht, worin $R_5$ H- oder Methyl und $Y_1$ O, S oder NH bedeuten und $X_1$ und $X_2$ die im Anspruch 1 angegebene Bedeutung haben.

5. Verfahren gemäss Anspruch 1, dadurch gekennzeichnet, dass man als metallabgebende Verbindung Salze des Nickels verwendet.

6. Verfahren gemäss Anspruch 1, dadurch gekennzeichnet, dass man als polares Lösungsmittel Dimethylformamid oder N-Methylpyrrolidon verwendet.

**Claims**

1. A process for the manufacture of a 1 : 1 metal complex of an azine of the formula

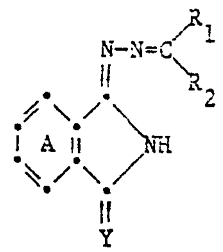

(I)

wherein $R_1$ is a hydrogen atom, an alkyl or aryl group, $R_2$ is an isocyclic or heterocyclic radical, both containing a hydroxyl or mercapto group adjacent to the azomethine group, Y is the radical of the formula

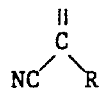

wherein R is a cyano group, an alkoxycarbonyl, alkylcarbamoyl or alkanoyl group, each of 2 to 6 carbon atoms, or is a benzoyl, carbamoyl, thiocarbamoyl or sulfamoyl group, a benzylcarbamoyl group, a phenylsulfamoyl group which is unsubstituted or substituted by halogen atoms or $C_1$-$C_4$ alkyl groups, or is a phenylsulfonyl group or a group of the formula

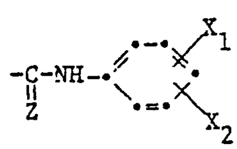

or

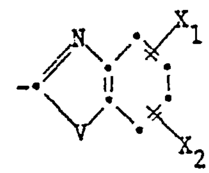

wherein Z is an oxygen or a sulfur atom, $X_1$ is a hydrogen, chlorine or bromine atom, a nitro, trifluoromethyl, carbamoyl or sulfamoyl group, an alkyl, alkoxy or alkylsulfamoyl group of 1 to 4 carbon atoms, an alkanoylamino, alkylcarbamoyl or alkoxycarbonyl group of 2 to 6 carbon atoms, a phenoxy, phenylcarbamoyl or phenylsulfamoyl group which is unsubstituted or substituted by chlorine or bromine atoms or methyl groups, and $X_2$ is a hydrogen, chlorine or bromine atom, an alkyl or alkoxy group of 1 to 4 carbon atoms, and V is O, S, or NH, and the ring A can contain substituents that do not confer solubility in water, which process comprises reacting a compound of the formula

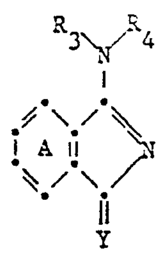

(II)

wherein $R_3$ is a hydrogen atom or an alkyl radical, $R_4$ is an alkyl, aryl or heteroaryl radical or the radical of the formula

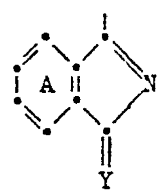

or $R_3$ and $R_4$, together with the nitrogen atom to which they are attached, form a 5- or 6-membered heterocyclic ring, and Y has the given meaning, with a hydrazone of the formula

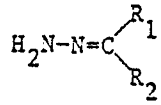

(III)

in the presence of a transition metal donor and in a polar organic solvent, in the temperature range from 100° to 200 °C.

2. A process according to claim 1, wherein the starting material is a compound of the formula (II), in which R is a carbamoyl or thiocarbamoyl group or a group of the formula

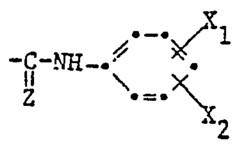

or

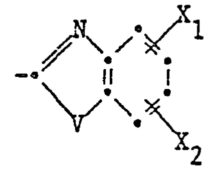

wherein Z is an oxygen or sulfur atom and $X_1$ and $X_2$ are as defined in claim 1.

3. A process according to claim 1, wherein the starting material is a compound of the formula (II), in which $R_3$—N—$R_4$ is the radical of the formula

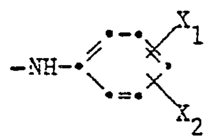

wherein $X_1$ and $X_2$ are as defined in claim 2.

4. A process according to claim 1, wherein the starting material is a hydrazone of the formula

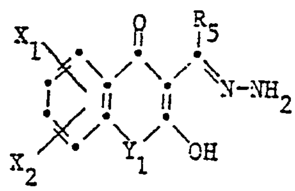

wherein $R_5$ is hydrogen or methyl and $Y_1$ is O, S or NH, and $X_1$ and $X_2$ are as defined in claim 1.

5. A process according to claim 1, wherein the metal donor is a nickel salt.

6. A process according to claim 1, wherein dimethyl formamide or N-methylpyrrolidone is used as polar solvent.

**Revendications**

1. Procédé de préparation de complexes de métaux 1 : 1 d'azines de formule

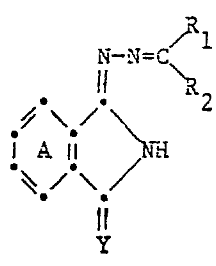

(I)

dans laquelle $R_1$ représente un atome d'hydrogène, un alkyle ou un aryle, $R_2$ un radical isocyclique ou hétérocyclique, les deux avec un groupe hydroxy ou mercapto voisin du groupe azométhinique, Y désigne un radical de formule

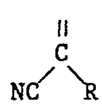

R étant un groupe cyano, alcoxycarbonyle, alkylcarbamoyle, alcanoyle en $C_2$ à $C_6$, un groupe benzoyle, carbamoyle, thiocarbamoyle ou sulfamoyle, un groupe benzylcarbamoyle, un groupe phénylsulfamoyle pouvant être éventuellement substitué par des atomes d'halogènes ou des alkyles en $C_1$ à $C_4$, ou encore un groupe phénylsulfonyle ou un groupe de formule

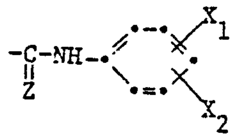

ou

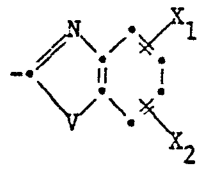

Z désignant un atome d'oxygène ou de soufre, $X_1$ un atome d'hydrogène, de chlore ou de brome, un groupe nitro, trifluorométhyle, carbamoyle ou sulfamoyle, un alkyle, un alcoxy ou un alkyl-sulfamoyle en $C_1$ à $C_4$, un groupe alcanoyl-amino, alkylcarbamoyle ou alcoxycarbonyle en $C_2$ à $C_6$, ou encore un groupe phénoxy, phénylcarbamoyle ou phénylsulfamoyle éventuellement substitué par des atomes de chlore ou de brome ou par des groupes méthylène, $X_2$ un atome d'hydrogène, de chlore ou de brome ou un alkyle ou un alcoxy en $C_1$ à $C_4$, et V l'oxygène, le soufre ou le groupe NH, et le cycle A ne peut pas avoir de substituants hydrosolubilisants, procédé caractérisé en ce que l'on fait réagir un composé de formule

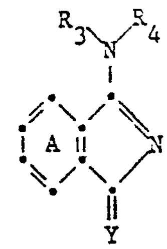

(II)

dans laquelle $R_3$ désigne un atome d'hydrogène ou un alkyle, $R_4$ un alkyle, un aryle ou un hétéroaryle ou encore un radical de formule

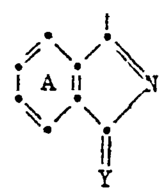

ou bien, $R_3$ et $R_4$ forment ensemble et avec l'atome d'azote un radical hétérocyclique pentagonal ou hexagonal, et Y a la signification donnée avec une hydrazone de formule

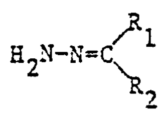

en présence d'un composé donnant un métal de transition, dans un solvant organique polaire et à des températures comprises entre 100 et 200 °C.

2. Procédé selon la revendication 1, caractérisé en ce qu'on part d'un composé de formule (II) dans lequel R est un groupe carbamoyle ou thiocarbamoyle ou un groupe de formule

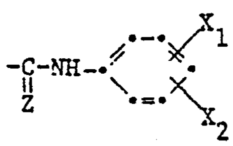

ou

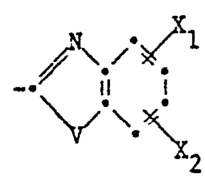

Z désignant un atome d'oxygène ou de soufre et les autres symboles ayant les significations données dans la revendication 1.

3. Procédé selon la revendication 1, caractérisé en ce que l'on part d'un composé de formule (II) dans lequel $R_3$—N—$R_4$ représente un radical de formule

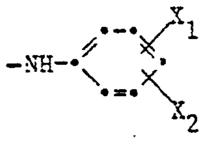

$X_1$ et $X_2$ ayant les significations données dans la revendication 1.

4. Procédé selon la revendication 1, caractérisé en ce que l'on part d'une hydrazone de formule

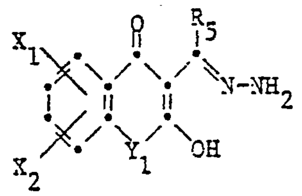

$R_5$ étant l'hydrogène ou un méthyle et $Y_1$ l'oxygène, le soufre ou le groupe NH, et $X_1$ et $X_2$ ayant les significations données dans la revendication 1.

5. Procédé selon la revendication 1, caractérisé en ce que l'on utilise un sel de nickel comme composé cédant le métal.

6. Procédé selon la revendication 1, caractérisé en ce que l'on utilise comme solvant polaire le diméthylformamide ou la N-méthylpyrrolidone.